# EUROPEAN PATENT APPLICATION

(11) **EP 4 579 238 A1**
(43) Date of publication of application: **02.07.2025**
(21) Application number: 24222917.7
(22) Date of filing: 23.12.2024
(51) Int. Cl.: G01N 33/52, G01N 33/543, G01N 33/94, A61K 38/13

(54) **REAGENT COMBINATION AND METHOD FOR DETECTING TARGET IMMUNOSUPPRESSANT IN WHOLE BLOOD WITHOUT CENTRIFUGATION**

(30) Priority: 31.12.2023 CN 202311861025
(71) Applicant: Shenzhen New Industries Biomedical Engineering Co., Ltd., Shenzhen, Guangdong 518122 (CN)
(72) Inventor: RAO, Jie, Guangdong, 518122 (CN); LI, Tinghua, Guangdong, 518122 (CN); ZHANG, Yun, Guangdong, 518122 (CN); LUO, Chun, Guangdong, 518122 (CN); ZHENG, Wendi, Guangdong, 518122 (CN); XU, Hong, Guangdong, 518122 (CN)
(74) Representative: Zacco GmbH

(57) **Abstract**

The present application relates to the field of in vitro detection technology, specifically to a reagent combination and a method for detecting a target immunosuppressant in whole blood without centrifugation. The present application proposes a reagent combination with low toxicity and safety, as well as a method for detecting a target immunosuppressant in whole blood. Using the method of the present application for immune detection, the detection results can maintain a high degree of consistency with mass spectrometry detection results, indicating that using the whole blood treatment agent of the present application to treat samples can fully release bound drugs, and the obtained free-state drugs can meet the requirements of immune detection. The detection method provided by the present application truly achieves detection without centrifugation, greatly improving detection efficiency.

## Description

### Technical Field

The present invention relates to the field of in vitro detection technology, specifically to a reagent combination and a method for detecting a target immunosuppressant in whole blood without centrifugation.

### Background

An immunosuppressant is a class of drugs with immunosuppressive effects, mainly used clinically for organ transplant rejection and autoimmune diseases.

After drugs are absorbed into the bloodstream through various routes of administration, they reach the site of action or the receptor site through the bloodstream. Free drugs in the blood diffuse into the extracellular fluid or further into the cell, and bind with the receptor to exert pharmacological effects. The distribution of drugs in tissues or their binding to receptors at the site of action is a reversible physiological and biochemical process that is in dynamic equilibrium. When drugs are evenly distributed in the body, although the drug concentrations in the blood and target sites are often not equal, for the vast majority of drugs, especially those transported passively in the body, the ratio of blood drug concentration to target drug concentration is constant. Due to the correlation between drug effects and blood drug concentration, the drug concentration in the blood can indirectly reflect the concentration of the drug at the receptor site. Therefore, measuring blood drug concentration has been widely accepted in clinical practice and has become the main method for monitoring therapeutic drugs.

On the other hand, in order to avoid organ transplant rejection, organ transplant recipients need to use immunosuppressants for life after transplantation, and these drugs have a narrow treatment range and significant side effects. When the effect of immunosuppressants is enhanced, the immune deficiency response is also enhanced, which weakens the patient's systemic immune function, reduces the patient's resistance to infections and tumors, and increases the incidence of infections and tumors. When the immunosuppressive effect weakens, it cannot effectively control and prevent rejection reactions, leading to the loss of function of grafts. Therefore, doctors need to directly participate in the pharmaceutical monitoring of patients after organ transplantation in order to improve the medication compliance, safety, effectiveness, rationality, and economy of organ transplant recipients.

Immunosuppressants commonly used for monitoring therapeutic drugs in clinical practice mainly include cyclosporine (CsA), tacrolimus, sirolimus, etc. Most of these drugs exist in the blood in a bound state. For example, CsA has a high binding rate with blood cells and plasma proteins in the blood, with 50% -75% being taken up by red blood cells and the rest distributed in the plasma, mostly bound to lipoproteins; after absorption of FK506, 75%-80% of FK506 in whole blood is presented in red blood cells, while FK506 in plasma mainly binds to plasma proteins, with a binding rate of over 98%; and 95% of sirolimus in the human body binds to red blood cells.

Due to the hydrophobic structure of immunosuppressants, they form relatively stable complexes with various macromolecules in blood and blood cells. Therefore, when performing whole blood detection, it is necessary to disrupt blood cells to release these stable complexes, and extract free immunosuppressants through displacement.

At present, chemiluminescence method is commonly used to detect immunosuppressants in blood or blood cells. Due to the influence of whole blood on the signal determination of luminescent groups in subsequent chemiluminescence methods, the traditional method is to first dissolve and centrifuge the blood, remove impurities, and release immunosuppressants from blood cells. However, the centrifugation process increases the workload of laboratory doctors, which is time-consuming and requires controlled conditions to ensure the quality of centrifuged samples. Additionally, toxic whole blood precipitants are often added, posing a safety hazard to laboratory personnel.

For example, publicly available methods for detecting immunosuppressants include the following steps:
taking whole blood samples, whole blood solubilizers, and whole blood precipitants (methanol and ethylene glycol solutions containing zinc sulfate), mixing them in a 2:1:4 ratio in XSYSTEMS centrifuge tubes, immediately vortexing the sample for 5-10 seconds, centrifuging for 4 minutes, transferring the supernatant to a pretreatment tube, and performing machine testing. In this solution, methanol and ethylene glycol are toxic, and the method also requires centrifugation, which is a cumbersome process.

Therefore, there is an urgent need to develop a reagent combination with low toxicity and safety, as well as a method for detecting an immunosuppressant based on this reagent combination. The reagent combination can be used for detecting the immunosuppressant in whole blood, and can meet the requirements of immune detection without the need for centrifugation.

### Summary

The present invention aims to at least partially solve one of the technical problems in related technologies. Therefore, one objective of the present invention is to propose a reagent combination with low toxicity and safety, as well as a method for detecting a target immunosuppressant in whole blood. Using the method of the present invention for immune detection, and after centrifugation, it can maintain a high degree of consistency with mass spectrometry detection results, indicating that bound state drugs in samples are fully released, and the obtained free-state drugs can meet the requirements of immune detection.

Therefore, the first aspect of the present invention provides a whole blood treatment agent. According to an embodiment of the present invention, the whole blood treatment agent comprises:
butanol and optionally saponin and/or ascorbic acid.

According to an embodiment of the present invention, when using the whole blood treatment agent to perform a treatment on a whole blood, a working concentration of the butanol is 0.1-1.0% v/v.

According to an embodiment of the present invention, when using the whole blood treatment agent to perform a treatment on a whole blood, a working concentration of the saponin is 0.3-3% w/v;
optionally, a working concentration of the ascorbic acid is 0.5-1.0% w/v.

The second aspect of the present invention provides a reagent combination. According to an embodiment of the present invention, the reagent combination comprises the whole blood treatment agent according to the first aspect and a buffer,
the buffer comprises at least one selected from the group consisting of a PBS buffer, a Tris buffer, and a MES buffer, and further comprises at least one selected from the group consisting of Tween-20, Triton, and a hydrophilic amino acid.

According to an embodiment of the present invention, the hydrophilic amino acid comprises at least one selected from the group consisting of arginine, asparagine, aspartic acid, glutamine, serine, threonine, cysteine, glutamic acid, lysine, and histidine.

According to an embodiment of the present invention, when the buffer contains Triton, a concentration of the Triton in the buffer is 0.065-0.3% v/v. The third aspect of the present invention provides a use of the whole blood treatment agent according to the first aspect and the reagent combination according to the second aspect for detecting a target molecule in a whole blood.

According to an embodiment of the present invention, the target molecule comprises an immunosuppressant.

According to an embodiment of the present invention, the immunosuppressant comprises at least one selected from the group consisting of cyclosporine, sirolimus, and FK506.

The fourth aspect of the present invention provides a method for detecting a target molecule in a whole blood. According to an embodiment of the present invention, the method comprises:
(1) using the whole blood treatment agent according to the first aspect to perform a treatment on a whole blood to be detected to obtain a treated sample to be detected; and
(2) detecting a target molecule in the treated sample to be detected based on an immunoassay method.

According to an embodiment of the present invention, the target molecule comprises an immunosuppressant.

According to an embodiment of the present invention, the immunosuppressant comprises at least one selected from the group consisting of cyclosporine, sirolimus, and FK506.

According to an embodiment of the present invention, a volume ratio of the whole blood to the whole blood treatment agent in step (1) is (1-50): (1-3).

The fifth aspect of the present invention provides a method for detecting a target immunosuppressant in a whole blood. According to an embodiment of the present invention, the method comprises:
1) providing a signal detection reagent and a magnetic carrier coated with a first antibody, wherein the signal detection reagent contains a signal generator connected to a second antibody, the first antibody can specifically recognize and bind a target molecule in a sample to be detected to form a complex of target molecule-first antibody, the target molecule in the complex induces the first antibody to form at least one new conformational epitope, and the second antibody can specifically bind to the at least one new conformational epitope; 2) using the whole blood treatment agent according to the first aspect to perform a treatment on a whole blood to be detected to obtain a treated sample to be detected;
3) contacting the treated sample to be detected with the magnetic carrier coated with the first antibody to obtain a complex of target immunosuppressant-first antibody-magnetic carrier;
4) contacting the complex of target immunosuppressant-first antibody-magnetic carrier with the signal detection reagent to obtain a complex of target immunosuppressant-first antibody-magnetic carrier-second antibody-signal generator; and
5) using an immunoassay method, performing signal detection on the complex of target immunosuppressant-first antibody-magnetic carrier-second antibody-signal generator obtained in 4) to detect the target immunosuppressant in whole blood.

According to an embodiment of the present invention, the affinity constant KD value between the second antibody and the target immunosuppressant-first antibody is 1×10⁻¹⁰-9×10⁻⁹.

According to an embodiment of the present invention, the magnetic carrier comprises any one of a magnetic bead or a microsphere.

According to an embodiment of the present invention, the magnetic carrier is a microsphere with a particle size of 0.3-9.0 µm.

According to an embodiment of the present invention, in a microsphere coated with a first molecule, the mass ratio of the first molecule to the microsphere is 1000: (8-20).

According to an embodiment of the present invention, the surface of the magnetic carrier is coated with a hydrophilic group.

According to an embodiment of the present invention, the hydrophilic group is selected from hydroxyl, carboxyl, and animo groups.

According to an embodiment of the present invention, a concentration of the hydrophilic group coated on the surface of the magnetic carrier is 0.5-2.0 mmol/g.

According to an embodiment of the present invention, the target immunosuppressant comprises at least one selected from the group consisting of cyclosporine, sirolimus, and FK506.

According to an embodiment of the present invention, the detection method further comprises providing a buffer, and contacting the treated sample to be detected with the magnetic carrier coated with the first antibody in the buffer.

According to an embodiment of the present invention, the buffer comprises at least one selected from the group consisting of a PBS buffer, a Tris buffer, and a MES buffer.

According to an embodiment of the present invention, the buffer further comprises at least one selected from the group consisting of Tween-20, Triton, and a hydrophilic amino acid.

According to an embodiment of the present invention, when the buffer contains Triton, the concentration of the Triton in the buffer is 0.065-0.3% v/v.

According to an embodiment of the present invention, the hydrophilic amino acid comprises at least one selected from the group consisting of arginine, asparagine, aspartic acid, glutamine, serine, threonine, cysteine, glutamic acid, lysine, and histidine.

According to an embodiment of the present invention, the signal generator comprises at least one selected from the group consisting of ABEl, an acridinium ester, luminol, isoluminol, AHEI, ITCI, and lucigenin.

The drug obtained from the sample treating scheme provided by the present invention can be subjected to immune detection. The drug can not only react with the first antibody, but also form the correct antigen recognition epitope which can be recognized by the second antibody after the first antibody reaction. Linear detection results can be obtained by detecting the formed sandwich complex. The detection scheme further provided by the present invention can reduce the difference in the detection results of whole blood before and after centrifugation, which is conducive to achieving detection without centrifugation and greatly improves the detection efficiency.

Additional aspects and advantages of the present invention will be partially presented in the following description, some of which will become apparent from the following description, or learned through practice of the present invention.

### Detailed Description of the Embodiments

Hereinafter, the examples of the present invention will be described in detail. The examples described below are exemplary and are intended to explain the present invention and are not to be construed as limiting the present invention.

It should be noted that the terms "first" and "second" are used for descriptive purposes only and are not to be construed as indicating or implying relative importance or implicitly indicating the number of technical features indicated. Thus, a feature defined with "first" or "second" may explicitly or implicitly includes one or more of the features. Further, in the description of the present invention, unless otherwise specified, the meaning of "a plurality" is two or more.

The endpoints of the ranges and any values disclosed herein are not limited to the precise range or value, and such ranges or values should be understood to include values approximating such ranges or values. For numerical ranges, the endpoints of each range, the endpoints of each range and the individual point values, and the individual point values, can be combined with each other to produce one or more new numerical ranges, and such numerical ranges are to be considered to be specifically disclosed herein.

In the present invention, unless otherwise specified, technical and scientific terms used herein have the meanings commonly understood by those skilled in the art. Moreover, protein and nucleic acid chemistry, molecular biology, related terms, and laboratory operating procedures used herein are widely used terms and routine procedures in the corresponding fields. For example, standard recombinant DNA and molecular cloning techniques used in the present invention are well-known to those skilled in the art and are more comprehensively described in the following documents: Sambrook, J., Fritsch, E.F. and Maniatis, T., Molecular Cloning: A Laboratory Manual; Cold Spring Harbor Laboratory Press: Cold Spring Harbor, 1989 (hereinafter referred to as "Sambrook").

The term "comprise" or "include" herein is an open expression, it means comprising the contents disclosed by the present invention, but don't exclude other contents.

The terms "optionally" or "optional" herein typically refer to the event or situation described later that may but may not necessarily occur, and the description includes situations where the event or situation occurs, as well as situations where the event or situation does not occur.

As used herein, the term "and/or" encompasses all combinations of items connected by this term and should be considered as separately listed herein. For example, "A and/or B" encompasses "A", "A and B", and "B". For example, "A, B and/or C" encompasses "A", "B", "C", "A and B", "A and C", "B and C", as well as "A and B and C".

According to a specific embodiment of the present invention, the present invention provides a whole blood treatment agent, comprising:
butanol and optionally saponin and/or ascorbic acid.

According to a specific embodiment of the present invention, the whole blood treatment agent provided by the present invention may include, for example, butanol alone, or butanol and saponin, or butanol and ascorbic acid. According to a preferred embodiment of the present invention, the whole blood treatment agent provided by the present invention includes butanol and saponin, or butanol and ascorbic acid. The whole blood treatment agent may also contain other ingredients, as long as the main substance that dissolves the cell debris of blood cells in whole blood is butanol and optionally saponin and/or ascorbic acid, and other auxiliary ingredients are also included in the scope of protection of the whole blood treatment agent of the present invention.

The inventors found that adding organic solvent butanol to the whole blood can further dissolve larger cell debris with the similarity-intermiscibility theory. For example, the addition amount is whole blood: butanol = 5:1-50:1. Phenol can also achieve similar technical effects, but due to its low solubility, it is difficult to prepare, so butanol is preferred. This treatment method can obtain free immunosuppressants, which can be used for immunological detection.

According to a specific embodiment of the present invention, when using the whole blood treatment agent to treat whole blood, a working concentration of the butanol is 0.1-1.0% v/v;
a working concentration of the saponin is 0.3-3.0% w/v; and
a working concentration of the ascorbic acid is 0.5-1 % w/v.

It should be noted that saponin or ascorbic acid can be directly mixed with butanol in solid form to form a whole blood treatment agent, or a whole blood treatment agent such as butanol, saponin, or ascorbic acid can be stored in solution. When used to detect a target molecule in whole blood, butanol, saponin, or ascorbic acid can be added to whole blood. The amount of butanol, saponin, or ascorbic acid added can be adjusted according to the concentration of butanol, saponin, or ascorbic acid solution and the volume of whole blood, as long as the final working concentration is within the above-mentioned range. For example, when the concentration of butanol solution stored is relatively high, the volume of butanol added to whole blood can be reduced accordingly.

According to a specific embodiment of the present invention, the present invention provides a reagent combination comprising the whole blood treatment agent as described above, and a buffer, the buffer comprises at least one selected from the group consisting of a PBS buffer, a Tris buffer, and a MES buffer, and further comprises at least one selected from the group consisting of Tween-20, Triton, and a hydrophilic amino acid.

According to a specific embodiment of the present invention, the hydrophilic amino acid comprises at least one selected from the group consisting of arginine, asparagine, aspartic acid, glutamine, serine, threonine, cysteine, glutamic acid, lysine, and histidine.

According to a specific embodiment of the present invention, when the buffer contains Triton, a concentration of the Triton in the buffer is 0.065-0.3% v/v.

The whole blood treatment agent or reagent combination provided by the present invention can be used for detecting a target molecule in whole blood. The target molecule comprises an immunosuppressant, such as cyclosporine, sirolimus, FK506, etc. Of course, other types of immunosuppressants that need to be detected clinically can also be detected using the whole blood treatment agent or reagent combination of the present invention.

According to a specific embodiment of the present invention, the present invention provides a method for detecting a target molecule in a whole blood, comprising:
(1) using the whole blood treatment agent as described above to performing a treatment on whole blood to be detected to obtain a treated sample to be detected; and
(2) detecting a target molecule in the treated sample to be detected based on an immunoassay method.

According to a specific embodiment of the present invention, the volume ratio of the whole blood to the whole blood treatment agent in step (1) is (1-50): (1-3). For example, when the whole blood treatment agent is 5% butanol or contains 5% butanol, the volume ratio of the whole blood to 5% butanol can be 5:1-50:1; when the whole blood treatment agent contains 30% saponin, the volume ratio of the whole blood to 30% saponin can be 10:1-100:1; and when the whole blood treatment agent contains 2% ascorbic acid, the volume ratio of the whole blood to 2% ascorbic acid can be 1:1-3:1.

An immunoassay method is a biochemical analysis method that utilizes immune reactions to detect and determine specific substances. With antibodies/antigens to detect certain substances, this method can quickly and accurately measure the content of substances, which can be proteins, nucleic acids, hormones, antigens, etc. In the present invention, the target molecule detected by the immunoassay method is an immunosuppressant, such as cyclosporine, sirolimus, FK506, etc. Any type of immunosuppressant known in the art that needs to be detected clinically in whole blood can be detected with the above-mentioned detection method for a target molecule in whole blood. Using the method of the present invention for immune detection, the detection results can maintain a high degree of consistency with mass spectrometry detection results, indicating that bound drugs in the sample are fully released, and the obtained free-state drugs can meet the requirements of immune detection. According to a specific embodiment of the present invention, the present invention provides a detection method for a target immunosuppressant in a whole blood, comprising:
1) providing a signal detection reagent and a magnetic carrier coated with a first antibody, wherein the signal detection reagent contains a signal generator connected to a second antibody, the first antibody can specifically recognize a target molecule in a sample to be detected and bind to form a complex of target molecule-first antibody, the target molecule in the complex induces the first antibody to form at least one new conformational epitope, and the second antibody can specifically bind to the at least one new conformational epitope;
(2) using the whole blood treatment agent as described above to performing a treatment on whole blood to be detected to obtain a treated sample to be detected;
3) contacting the treated sample to be detected with the magnetic carrier coated with the first antibody to obtain a complex of target immunosuppressant-first antibody-magnetic carrier;
4) contacting the complex of target immunosuppressant-first antibody-magnetic carrier with the signal detection reagent to obtain a complex of target immunosuppressant-first antibody-magnetic carrier-second antibody-signal generator; and
5) using an immunoassay method, performing optical signal detection on the complex of target immunosuppressant-first antibody-magnetic carrier-second antibody-signal generator obtained in 4) to detect the target immunosuppressant in whole blood.

According to a specific embodiment of the present invention, a reagent required for immunological testing include a magnetic ball reagent, an ABEl reagent, and a buffer, wherein the magnetic ball is coated with a CsA antibody, and the ABEl reagent contains another antibody that specifically recognizes a CsA-CsA antibody complex. The buffer contains Triton.

When detecting, the sample is mixed with the whole blood treatment agent (containing saponin/ascorbic acid/butanol), and then mixed with the magnetic ball reagent and the buffer. After sufficient reaction, the mixture is washed, and then the ABEl reagent is added. After sufficient reaction, the mixture is washed, and the amount of CsA in the sample is reflected by detecting a ternary complex.

Through experimental verification, it has been found that CsA obtained through the above-mentioned sample treating can not only react with the first antibody, but also form the correct antigen recognition epitope after the first antibody reaction, which can be recognized by the complex antibody (the second antibody). Linear detection results can be obtained by detecting the formed sandwich complex.

Using the above-mentioned methods to treat and detect samples, there is still a small difference in the detection results before and after centrifugation, and further simplification of the detection steps for the immunosuppressant is needed.

Therefore, another aspect of the present invention provides an immunosuppressant detection reagent system method that can reduce the difference in detection results before and after centrifugation, which is beneficial for achieving immunosuppressant detection without centrifugation.

The inventors also made the following specific optimizations regarding the reagent components:
① Magnetic ball: the inventors found the further optimized performance of the magnetic ball, such as increasing the hydrophilic groups on the surface of the magnetic ball (500-2000 mmol/g), such as carboxyl, hydroxyl, and animo groups.
(2) Buffer: adding Tween-20 increases the viscosity of the reaction system, reduces the settling speed of the cell debris, and accelerates the further separation of the magnetic ball and the cell debris.
(1) Buffer: Adding hydrophilic substances such as amino acids (arginine, lysine, asparagine, histidine), and surfactants (Tween, Brij-35), etc., can reduce the binding of the cell debris to the magnetic ball.

Through experimental verification, it has been proven that optimizing the above-mentioned reagent components can solve the problem of inconsistent results before and after centrifugation in sandwich method detection. The inventors speculate that it is possible that the broken membrane debris of blood cells are hydrophobic substances that will non-specifically adsorb onto the magnetic ball, causing FK506/CsA small molecules to be unable to bind to the first antibody, or that the hydrophobic substance blocks the recognition epitope of the complex antibody after CsA binds to the first antibody.

According to a specific embodiment of the present invention, the affinity constant KD value of the second antibody is 1×10⁻¹⁰-9×10⁻⁹.

According to a specific embodiment of the present invention, the magnetic carrier comprises any one of a magnetic bead or a microsphere. When the magnetic carrier is a microsphere, the preferred particle size of the microsphere is 0.3-9.0 µm.

According to a specific embodiment of the present invention, a mass ratio of the first molecule to the microsphere in a microsphere coated with a first molecule is 1000: (8-20). For example, a mass ratio of the first molecule to the microsphere in a microsphere coated with a first molecule is 1 mg: 8 ug-1 mg: 20 ug.

According to a preferred embodiment of the present invention, the surface of the magnetic carrier is coated with a hydrophilic group. According to a specific embodiment of the present invention, the hydrophilic group is selected from hydroxyl, carboxyl, and animo groups. Of course, in addition to these hydrophilic groups, the surface of the magnetic carrier of the present invention can also be coated with other types of hydrophilic groups, preferably hydroxyl groups.

According to a preferred embodiment of the present invention, a concentration of the hydrophilic group coated on the surface of the magnetic carrier is 0.5-2.0 mmol/g. A concentration here refers to the average coating of 0.5-2 mmol of the hydrophilic group on the surface of each 1 g of the magnetic carrier.

According to a specific embodiment of the present invention, the target immunosuppressant comprises at least one selected from the group consisting of cyclosporine, sirolimus, and FK506.

According to a specific embodiment of the present invention, step 3) further comprises contacting the treated sample to be detected with the magnetic carrier coated with the first antibody in the buffer.

According to a specific embodiment of the present invention, the buffer comprises at least one selected from the group consisting of a PBS buffer, a Tris buffer, and a MES buffer.

According to a specific embodiment of the present invention, the buffer further comprises at least one selected from the group consisting of Tween-20, Triton, and a hydrophilic amino acid.

According to a specific embodiment of the present invention, when the buffer contains Triton, a concentration of the Triton in the buffer is 0.065-0.30% v/v.

According to a specific embodiment of the present invention, the hydrophilic amino acid comprises at least one selected from the group consisting of arginine, asparagine, aspartic acid, glutamine, serine, threonine, cysteine, glutamic acid, lysine, and histidine.

According to a specific embodiment of the present invention, the signal generator comprises at least one selected from the group consisting of ABEl, an acridinium ester, luminol, isoluminol, AHEI, ITCI, and lucigenin. Preferably, the signal generator is ABEl.

The following will explain the solution of the present invention in conjunction with the examples. Those skilled in the art will understand that the following examples are only used to illustrate the present invention and should not be considered as limiting the scope of the present invention. If specific technology or conditions are not specified in the examples, follow the technology or conditions described in the documents in the art or follow the product manual. The reagents or instruments used without specifying the manufacturer are conventional products that can be obtained through commercial purchase.

### Antibody binding affinity validation

BIAcore technology was used to detect the binding affinity of CsA antibodies and CsA-CsA antibody complexes with CsA complex antibodies (as shown in Table 1). The experimental process was carried out according to the BiacoreR 3000 and Biacore AB manual. The equilibrium dissociation constant Kd (M) represents the degree of dissociation of binding affinity in equilibrium state, and the smaller Kd (M), the stronger the affinity.

**Table 1**

| | | Ka (M⁻¹s⁻¹) | Kd (s⁻¹) | Kd (M) |
|---|---|---|---|---|
| CsA complex antibody | CsA antibody | 2.56×10² | 4.73×10⁻³ | 1.85×10⁻⁵ |
| | CsA-CsA antibody complex | 4.49×10² | 9.84×10⁻⁷ | 2.19×10⁻⁹ |

**Table 2 shows the binding affinity of FK 506 antibody and FK 506-FK 506 antibody complex with FK 506 complex antibody detected using the same technique as described above.**

| Table 2 | | Ka (M⁻¹s⁻¹) | Kd (s⁻¹) | Kd (M) |
|---|---|---|---|---|
| FK 506 complex antibody | FK 506 antibody | 3.07×10² | 1.64×10⁻³ | 2.49×10⁻⁵ |
| | FK 506- FK 506 antibody complex | 9.61×10² | 1.25×10⁻⁷ | 1.30×10⁻¹⁰ |

### Example 1 Detection of immunosuppressants

### 1. 1. Reagents used for detecting CsA

(1) Magnetic ball reagents: magnetic balls coated with the first antibody that specifically recognizes CsA, the ratio of magnetic balls to antibody feeding: 1 mg: 12 ug, and the magnetic ball suspension concentration: 20mg/mL;
(2) Labeling reagents: the second antibody that specifically recognizes CsA and CsA antibody complexes is linked to ABEl, and the feeding ratio of ABEl and the second antibody: 0.5 mg: 12.5 ug;
whole blood treatment agent: as shown in Table 1.

Buffer: MES buffer system, containing 0.5% Triton.

### Sample addition process:

According to the addition ratio shown in Table 3, the whole blood and the whole blood treatment agent were mixed, and detection was performed on the MAGLUMI X8 chemiluminescence immunoassay analyzer of Shenzhen New Industry Biomedical Engineering Co., Ltd. The instrument sampling process parameters include: 10 µL of sample mixed with the whole blood treatment agent + 20 uL of magnetic ball + 200 ul of buffer, incubating for 4 minutes, washing 3 times + 150 uL of ABEl, incubating for 6 minutes, washing 3 times, and detecting.

**Table 3 Optimization scheme for CsA whole blood treatment agent**

| Examples | Testing items | components adding to the whole blood treatment agents | Whole blood: whole blood treatment agent |
|---|---|---|---|
| | | | Adding proportion |
| Example 2 | | 30% saponin | 100:1 |
| Example 3 | | | 50:1 |
| Example 4 | | | 10:1 |
| Example 5 | | 2% ascorbic acid | 3:1 |
| Example 6 | | | 2:1 |
| Example 7 | CsA | | 1:1 |
| Example 8 | | 5% butanol | 50:1 |
| Example 9 | | | 25:1 |
| Example 10 | | | 10:1 |
| Example 11 | | | 5:1 |
| Example 12 | | 30% saponin + 5% butanol | 50:1:2 |
| Example 13 | | 2% ascorbic acid + 5% butanol | 5:5:1 |

Furthermore, the magnetic ball reagents and labeling reagents for the above-mentioned detection reagents were optimized as shown in Table 4.

**Table 4 Optimization scheme for CsA detection reagents**

| Examples | Items | Whole blood treatment | Optimization of reagent components | Adding proportion |
|---|---|---|---|---|
| Example 14 | | | Hydrophilic groups on magnetic spheres (umol/g) | 2000 |
| Example 15 | | | | 1000 |
| Example 16 | | | | 500 |
| Example 17 | | | Adding T-20 to buffer | 0.05% |
| Example 18 | CsA | Whole blood: saponin: butanol = 50:1:2 | | 0.02% |
| Example 19 | | | Adding hydrophilic substance arginine to buffer | 7% |
| Example 20 | | | | 5% |
| Example 21 | | | | 3% |
| Example 22 | | | Hydrophilic groups on magnetic spheres (umol/g) | 1000+ 0.1% + 5% |
| | | | and adding T-20 and hydrophilic substance arginine to buffer | |

### 2. 2. Reagents used for detecting FK 506

(1) Magnetic ball reagents: magnetic balls coated with the first antibody that specifically recognizes FK506, the ratio of magnetic balls to antibody feeding: 1 mg: 10 ug, and the magnetic ball suspension concentration (Tris buffer): 20 mg/mL;
(2) Labeling reagents: the second antibody that specifically recognizes FK506 and FK506 antibody complexes is linked to ABEl, and the feeding ratio of ABEl and the complex antibody: 0.5 mg: 5 ug.

Buffer: MES buffer system, containing 0.5% Triton.

### Sample addition process:

According to the addition ratio shown in Table 5, whole blood and whole blood treatment agent were mixed. 10 µL of the sample mixed with the whole blood treatment agent, + 100 µL of buffer + 20 uL of magnetic ball, incubating for 6 minutes, + 150 µL of ABEl, incubating for 9 minutes, washing 3 times, and detecting.

**Table 5: Optimization scheme for FK506 whole blood treatment agent and detection reagents**

| Examples | Items | Whole blood treatment | Optimization of reagent components | Adding proportion |
|---|---|---|---|---|
| Example 23 | FK506 | Whole blood: saponin: butanol = 50:1:2 | Hydrophilic groups on magnetic spheres (umol/g) | 1000 + 0.1% + 5% |
| | | | and adding T-20 and hydrophilic substance arginine to buffer | |
| Example 24 | | Whole blood: saponin = 50:1 | - | - |

### Validation experiment 1: comparison of potency deviation of CsA samples before and after centrifugation

CsA samples: 10 samples were obtained by adding 1500, 1352, 1204, 1056, 908, 760, 612, 464, 316, 168, and 20 ng/mL of CsA standard to whole blood samples (normal human blood without medication).

Calculation method for deviation:
Deviation% = potency after centrifugation/potency before centrifugation - 1

Average deviation% = the average of the deviations% of all samples.

Table 6 shows the average deviation of CsA sample detection results before and after centrifugation in Examples 2-22:

**Table 6**

| Comparison of CsA samples before and after centrifugation | |
|---|---|
| Examples | Average deviation |
| Example 2 | 70.81% |
| Example 3 | 62.65% |
| Example 4 | 79.54% |
| Example 5 | 65.82% |
| Example 6 | 60.54% |
| Example 7 | 79.23% |
| Example 8 | 53.13% |
| Example 9 | 38.20% |
| Example 10 | 40.68% |
| Example 11 | 58.20% |
| Example 12 | 20.35% |
| Example 13 | 35.64% |
| Example 14 | 18.19% |
| Example 15 | 15.64% |
| Example 16 | 20.98% |
| Example 17 | 15.02% |
| Example 18 | 19.53% |
| Example 19 | 13.43% |
| Example 20 | 10.69% |
| Example 21 | 17.35% |
| Example 22 | 5.61% |

### Validation experiment 2: comparison of potency deviation of FK 506 samples before and after centrifugation

FK 506 samples: 10 samples were obtained by adding 50, 45.05, 40.1, 35.15, 30.2, 25.25, 20.3, 15.35, 10.4, 5.45, and 0.5 ng/mL of FK 506 standard to whole blood samples (normal human blood without medication).

According to the same deviation calculation method as in validation experiment 1, the average deviation of the FK 50 sample detection results before and after centrifugation in Example 23 and Comparative example 1 was calculated, as shown in Table 7:

**Table 7**

| Comparison of FK 506 samples before and after centrifugation | |
|---|---|
| Examples | Average deviation |
| Example 23 | 1.96% |
| Example 24 | 68.51% |

The data from Validation experiments 1 and 2 demonstrates that:
The detection results of Example 2-4 showed that adding 30% saponin during sample pretreatment resulted in the best effect when the ratio of whole blood to 30% saponin was 10:1-100:1. At this time, the average deviation of potency before and after centrifugation was 70.81%, 62.65%, and 79.54%, respectively; however, there was a significant deviation before and after centrifugation, which cannot guarantee the consistency of centrifugal and non-centrifugal detection results.

The detection results of Examples 5-7 showed that adding 2% ascorbic acid during sample pretreatment resulted in the best effect when the ratio of whole blood to 2% ascorbic acid was 1:1-3:1. At this time, the average deviation of potency before and after centrifugation was 65.82%, 60.54%, and 79.23%, respectively; compared to Examples 2-4, the average deviation before and after centrifugation exceeded 60%, indicating a significant deviation issue that cannot guarantee the consistency of centrifugal and non-centrifugal detection results.

The detection results of Example 8-11 showed that adding 5% butanol during the sample pretreatment process resulted in the best effect when the ratio of whole blood to butanol was 5:1-50:1. At this time, the average deviation of potency before and after centrifugation was 53.13%, 38.20%, 40.68%, and 58.20%, respectively; compared to the example where only saponin was added, the deviation before and after centrifugation was significantly reduced, which can guarantee the relative consistency of centrifugal and non-centrifugal detection results.

The detection results of Examples 12-13 showed that when evaluating the better resulted in the previous examples, when the ratio of whole blood: 30% saponin: 5% butanol is 50:1:2, the average deviation of potency before and after centrifugation was 20.35%; when the ratio of whole blood: 2% ascorbic acid: 5% butanol was 5:5:1, the average deviation of potency before and after centrifugation was 35.64%. Compared to the example where only saponin was added and Examples 5-7, the deviation before and after centrifugation was further reduced, which can guarantee the relative consistency of centrifugal and non-centrifugal detection results.

The detection results of Examples 14-16 showed that the average deviation of potency before and after centrifugation was 18.19%, 15.64%, and 20.98%, respectively, when the number of hydrophilic groups crosslinked on the magnetic ball was increased to 500, 1000, and 2000 umol/g; therefore, compared to Example 12, the consistency between centrifugal and non-centrifugal detection results can be further improved.

The detection results of Examples 17 and 18 showed that 0.05% and 0.02% Tween-20 was attempted to be added to the buffer. At this time, the average deviation of potency before and after centrifugation was 15.02% and 19.53%, respectively; compared to Example 12, it is indicated that adding Tween-20 can further improve the consistency between centrifugal and non-centrifugal detection results.

The detection results of Examples 19-21 showed that when different amounts of hydrophilic substance arginine were attempted to be added to the buffer, the average deviation of potency before and after centrifugation was 13.43%, 10.69%, and 17.35% at the addition amounts of 3%, 5%, and 7%, respectively; compared to Example 12, it is indicated that adding hydrophilic substance arginine to the buffer can further improve the consistency of centrifugal and non-centrifugal detection results.

The detection results of Example 22 showed that the average deviation of potency before and after centrifugation was 5.61% when attempting to mix the optimal effects of the above-mentioned examples for evaluation, further solving the problem of the difference in potency before and after centrifugation in whole blood treatment samples detected by sandwich method reagents.

The detection results of Example 23 showed that the potency deviation of FK 506 before and after centrifugation in Example 24 was 68.51%. Applying the optimized CsA scheme to FK 506, the average potency deviation before and after centrifugation is 1.96%, further solving the problem of differences in whole blood treatment samples before and after centrifugation detected by sandwich method reagents.

Validation experiment 3: consistency comparison between results before and after centrifugation and mass spectrometry detection results

CsA and FK 506: mass spectrometry detection method. The whole blood sample to be detected was added with internal standard solution, red blood cell lysis solution, protein precipitant, and magnetic bead solution, shaken, and then subjected to magnetic separation. After magnetic separation, the supernatant was obtained, and the sample processing process was completed.

Specific experimental parameters:
(1) whole blood sample: an addition ratio of internal standard solution: red blood cell lysis solution: protein precipitant: magnetic bead solution = 50:1:1:30:10, and after magnetic separation, the supernatant was collected;
(2) the chromatographic conditions for liquid chromatography tandem mass spectrometry were as follows: mobile phase A and mobile phase B, the elution procedure was as follows: from 0 to 0.5 minutes, the volume fraction of mobile phase B was 100%; from 0.5 to 1.5 minutes, the volume fraction of mobile phase B was 80%-100%; from 1.5 to 2.0 minutes, the volume fraction of mobile phase B was 100%; and from 2.0 to 3.0 minutes, the volume fraction of mobile phase B was 50%;
(3) chromatographic conditions: the analytical column was C18 column; flow rate: 0.5 mL/min, column temperature: 45°C; injection chamber temperature: 2-8°C; injection volume: 10 uL; and
(4) mass spectrum conditions: electrospray ion source (ESI), positive ion MRM scanning; the specific parameters of the positive ion MTM scanning are: ionization voltage 5500 (V), temperature 480°C, curtain gas 30 psi, collision gas 6 psi, spray gas 45 psi, auxiliary heating gas 45 psi.

Validation sample: 100 whole blood samples using CsA drug.

The verification detection results are shown in Tables 8, 9, and 10:

**Table 8**

| | After centrifugation | |
|---|---|---|
| | R² | K |
| Example 2 | 0.971 | 1.016 |
| Example 3 | 0.956 | 0.957 |
| Example 4 | 0.968 | 0.960 |
| Example 5 | 0.906 | 0.980 |
| Example 6 | 0.972 | 1.045 |
| Example 7 | 0.963 | 1.013 |
| Example 8 | 0.990 | 0.998 |
| Example 9 | 0.953 | 1.020 |
| Example 10 | 0.949 | 0.972 |
| Example 11 | 0.954 | 1.022 |
| Example 12 | 0.954 | 0.993 |
| Example 13 | 0.986 | 1.045 |
| Example 14 | 0.978 | 1.025 |
| Example 15 | 0.986 | 1.048 |
| Example 16 | 0.923 | 1.039 |
| Example 17 | 0.986 | 1.042 |
| Example 18 | 0.910 | 0.976 |
| Example 19 | 0.934 | 0.991 |
| Example 20 | 0.908 | 0.994 |
| Example 21 | 0.976 | 1.019 |
| Example 22 | 0.984 | 0.992 |

**Table 9**

| CsA sample comparison | | | | |
|---|---|---|---|---|
| | Before centrifugation | | After centrifugation | |
| | R² | K | R² | K |
| Example 3 | 0.693 | 0.619 | 0.956 | 0.957 |
| Example 22 | 0.986 | 1.045 | 0.984 | 0.992 |

**Table 10**

| FK 506 sample comparison | | | | |
|---|---|---|---|---|
| | Before centrifugation | | After centrifugation | |
| | R² | K | R² | K |
| Example 23 | 0.991 | 0.986 | 0.991 | 1.003 |
| Example 24 | 0.269 | 0.157 | 0.985 | 1.012 |

The results in Table 8 indicated that: the optimization scheme of the whole blood treatment agent using the scheme of the present invention, or further optimization of the magnetic ball reagent, buffer and other reagent components based on the optimization of the whole blood treatment agent, resulted in a correlation of 0.9 or above with the detection results of mass spectrometry after centrifugation. Therefore, the above-mentioned scheme can fully release bound drugs in the sample, and the released free state drugs can meet the requirements of immune detection. Compared to the toxic whole blood treating scheme in existing technology centers, this scheme provides a more environmentally friendly and safe alternative.

The results of Tables 9 and 10 indicated that:
Example 24 of FK 506 item has a mass spectrometry correlation of only 0.269 before sample pretreatment and centrifugation, but the correlation increases to 0.985 after centrifugation. The potency deviation before and after centrifugation was significant, but there was a significant improvement after centrifugation.

The detection results of Example 3 showed that when a certain amount of saponin was added during the sample pretreatment process, and the addition ratio of whole blood: saponin was 50:1, a mass spectrometry correlation before and after centrifugation was 0.693 and 0.936, respectively; the correlation between the detection results after centrifugation and mass spectrometry reached over 90%, indicating that the sample treatment method of Example 3 (the addition ratio of whole blood: saponin wais in the range of 10:1-100:1) can meet the requirements of immune detection after centrifugation. However, the detection results of Example 22 showed that the optimal effect of the above-mentioned examples was mixed and evaluated. At this time, the mass spectrometry correlation before and after centrifugation was comparable, with 0.986 and 0.984, respectively, completely solving the problem of differences in the sandwich method reagent detection of whole blood treatment samples before and after centrifugation.

The detection results of Example 23 showed that: the optimized scheme of CsA was applied to FK 506, and the mass spectrometry correlation before and after centrifugation was comparable, with 0.991 and 0.991, respectively, completely solving the problem of differences in the sandwich method reagent detection of whole blood treatment samples before and after centrifugation.

In the description of this description, the reference to the terms "one embodiment", "some embodiments", "examples", "specific examples", "some embodiments" or "some examples" means that the specific features, structures, materials or characteristics described in conjunction with the embodiment or example are included in at least one embodiment or example of the present invention. In this description, the schematic expressions of the above-mentioned terms do not necessarily refer to the same embodiments or examples. Moreover, the specific features, structures, materials, or characteristics described can be combined in any one or more embodiments or examples in an appropriate manner. In addition, those skilled in the art can incorporate and combine the different embodiments or examples described in this description, as well as the features of different embodiments or examples, without conflicting with each other.

Although the embodiments of the present invention have been shown and described above, it can be understood that the above-mentioned embodiments are exemplary and should not be construed as limiting the present invention. Those skilled in the art can make changes, modifications, substitutions, and variations to the above-mentioned embodiments within the scope of the present invention.

## Claims

1. A whole blood treatment agent for detecting a target molecule in a whole blood, wherein the whole blood treatment agent comprises:
butanol and optionally saponin and/or ascorbic acid;
wherein the target molecule comprises an immunosuppressant.

2. The whole blood treatment agent according to claim 1, wherein when using the whole blood treatment agent to perform a treatment on a whole blood, a working concentration of the butanol is 0.1-1% v/v;
optionally, a working concentration of the saponin is 0.3-3% w/v; and
optionally, a working concentration of the ascorbic acid is 0.5-1% w/v.

3. The whole blood treatment agent according to claim 1, wherein the immunosuppressant comprises at least one selected from the group consisting of cyclosporine, sirolimus, and FK506.

4. A reagent combination for detecting a target molecule in a whole blood, wherein the reagent combination comprises the whole blood treatment agent according to any one of claims 1 - 3, and a buffer,
the buffer comprises at least one selected from the group consisting of a PBS buffer, a Tris buffer, and a MES buffer, and further comprises at least one selected from the group consisting of Tween-20, Triton, and a hydrophilic amino acid.

5. The reagent combination according to claim 4, wherein the hydrophilic amino acid comprises at least one selected from the group consisting of arginine, asparagine, aspartic acid, glutamine, serine, threonine, cysteine, glutamic acid, lysine, and histidine.

6. A method for detecting a target molecule in a whole blood, wherein the method comprises:
(1) treating a whole blood to be detected with the whole blood treatment agent according to any one of claims 1 - 3 to obtain a treated sample to be detected; and
(2) detecting a target molecule in the treated sample to be detected based on an immunoassay method;
wherein the target molecule comprises an immunosuppressant.

7. The method according to claim 6, wherein the immunoassay method comprises applying a solid-phrase carrier coated by antigens or antibodies, and the solid-phrase carrier is a magnetic carrier;
optionally, the magnetic carrier comprise any one of a magnetic bead or a microsphere.

8. The method according to claim 6, wherein the volume ratio of the whole blood to the whole blood treatment agent in step (1) is (1-50): (1-3).

9. The method according to claim 6, wherein the method comprises:
1) providing a signal detection reagent and a magnetic carrier coated with a first antibody, wherein the signal detection reagent comprises a signal generator connected to a second antibody, the first antibody can specifically recognize and bind a target immunosuppressant in a sample to be detected to form a complex of target molecule-first antibody, the target molecule in the complex induces the first antibody to form at least one new conformational epitope, and the second antibody can specifically bind to the at least one new conformational epitope;
2) using the whole blood treatment agent according to any one of claims 1 - 3 to treat the whole blood to be detected to obtain the treated sample to be detected;
3) contacting the treated sample to be detected with the magnetic carrier coated with the first antibody to obtain a complex of target immunosuppressant-first antibody-magnetic carrier;
4) contacting the complex of target immunosuppressant-first antibody-magnetic carrier with the signal detection reagent to obtain a complex of target immunosuppressant-first antibody-magnetic carrier-second antibody-signal generator; and
5) using an immunoassay method, performing signal detection on the complex of target immunosuppressant-first antibody-magnetic carrier-second antibody-signal generator obtained in 4) to detect the target immunosuppressant in the whole blood.

10. The method according to claim 9, wherein the affinity constant KD value of the second antibody is 1×10⁻¹⁰- 9×10⁻⁹;
optionally, the magnetic carrier comprises any one of a magnetic bead or a microsphere; optionally, the magnetic carrier is a microsphere with a particle size of 0.3-9.0 µm;
optionally, in a microsphere coated with a first molecule, a mass ratio of the first molecule to the microsphere is 1000: (8-20);
optionally, the surface of the magnetic carrier is coated with a hydrophilic group;
optionally, the hydrophilic group is selected from hydroxyl, carboxyl, and animo groups; and
optionally, a concentration of the hydrophilic group coated on the surface of the magnetic carrier is 0.5-2 mmol/g.

11. The method according to claim 9, wherein the target immunosuppressant comprises at least one selected from the group consisting of cyclosporine, sirolimus, and FK506.

12. The method according to claim 9, wherein the method further comprises providing a buffer, wherein the treated sample to be detected is in contact with the magnetic carrier coated with the first antibody in the buffer; optionally, the buffer comprises at least one selected from the group consisting of a PBS buffer, a Tris buffer, and a MES buffer.

13. The detection method according to claim 9, wherein the signal generator comprises at least one selected from the group consisting of ABEl, an acridinium ester, luminol, isoluminol, AHEI, ITCI, and lucigenin.

14. The method according to claim 12, wherein the buffer further comprises at least one selected from the group consisting of Tween-20, Triton, and a hydrophilic amino acid; and
optionally, the hydrophilic amino acid comprises at least one selected from the group consisting of arginine, asparagine, aspartic acid, glutamine, serine, threonine, cysteine, glutamic acid, lysine, and histidine.

15. The method according to claim 12, wherein when the buffer comprises Triton, a concentration of the Triton in the buffer is 0.065-0.3% v/v.
